Europäisches Patentamt

European Patent Office

Office européen des brevets

(11) Veröffentlichungsnummer: **0 278 215**
**A1**

## EUROPÄISCHE PATENTANMELDUNG

(21) Anmeldenummer: **88100088.9**

(51) Int. Cl.⁴: **A61B 5/04**

(22) Anmeldetag: **07.01.88**

(30) Priorität: **22.01.87 CH 224/87**

(43) Veröffentlichungstag der Anmeldung:
**17.08.88 Patentblatt 88/33**

(84) Benannte Vertragsstaaten:
**AT BE CH DE FR GB IT LI NL SE**

(71) Anmelder: **WILLI STUDER AG Fabrik für elektronische Apparate**
**Althardstrasse 30**
**CH-8105 Regensdorf ZH(CH)**

(72) Erfinder: **Schmid, Johann Jakob**
**Rosengartenstrasse 33**
**CH-8107 Buchs ZH(CH)**
Erfinder: **Thie, Werner**
**Lindenstrasse 27**
**CH-8400 Winterthur(CH)**

(54) **Verfahren und Vorrichtung zur Verarbeitung von Signalen aus einem physiologisch erzeugten elektrischen Feld.**

(57) Verfahren und Vorrichtung zur Kardiogoniometrie Bei einem solchen Verfahren oder einer solchen Vorrichtung, wo elektrische Signale als Potentiale in einem physiologisch erzeugten elektrischen Feld abgegriffen werden, wird ein Vektor ermittelt, der den momentanen Zustand eines elektrischen Dipols darstellt. Um die Bewegung des Vektors weitergehend erfassen und auslegen zu koennen, wird auch der zeitliche Verlauf des Vektors ermittelt. Dazu dient die Ermittlung von Differenzvektoren, von Flaechen (18) die der Vektor ueberstreicht, von Schlingen (15, 16) die der Vektor bildet und von Projektionen (15', 16') dieser Schlingen in eine Ebene (10).

Fig.5

## Verfahren und Vorrichtung zur Verarbeitung von Signalen aus einem physiologisch erzeugten elektrischen Feld

Die Erfindung betrifft ein Verfahren und eine Vorrichtung zur Verarbeitung von elektrischen Signalen, die als Potentiale in einem physiologisch erzeugten elektrischen Feld abgegriffen werden.

Aus der US Patentschrift Nr 4,569,357 ist ein solches Verfahren und eine Solche Vorrichtung bekannt, die es erlauben, die elektrischen Signale so abzugreifen und zu verarbeiten, dass daraus eine raeumliche Darstellung eines Vektors gewonnen werden kann, der das elektrische Feld darstellt, wie es vom Herzen eines Lebewesens erzeugt wird. Dabei wird der maximale Betrag und der Zeitpunkt zu dem der maximale Betrag auftritt, sowie die Richtung des Vektors zu diesem Zeitpunkt bestimmt.

Der Nachteil dieses bekannten Vefahrens und dieser Vorrichtung besteht darin, dass das Verhalten des genannten Vektors innerhalb einer Periode und innerhalb von mehreren Perioden nur ungenuegend erfasst wird. Damit verzichtet man auf wesentliche Hinweise ueber die Taetigkeit des Herzens, die durch eine genauere und vollstaendigere Erfassung des Verhaltens des Vektors zu gewinnen waeren.

Die Erfindung, wie sie in den Patentanspruechen gekennzeichnet ist, loest die Aufgabe, ein Verfahren und eine Vorrichtung zu schaffen, mit denen diese Nachteile vermieden werden koennen und mit denen die Bewegung des Vektors im Raum weitergehend erfasst und ausgelegt werden kann.

Die durch die Erfindung erreichten Vorteile sind insbesondere darin zu sehen, dass der zeitliche Verlauf des Vektors so erfasst wird, dass daraus weitere Groessen oder Masszahlen abgeleitet werden koennen. Der Vergleich solcher Groessen aus aufeinanderfolgenden Perioden und der Vergleich solcher Groessen mit entsprechenden Groessen die aus anderen Feldern der gleichen Art ermittelt werden, erlauben es, den Zustand des Herzens eines bestimmten Subjektes viel besser zu erfassen. Durch die gemaess der Erfindung vorgeschlagene qualitativ numerische Erfassung von Vektorschleifen wird es ermoeglicht, einzelne Vektorschleifen auf objektivere Art und Weise miteinander zu vergleichen. Dies geschieht indem aus jeder Vektorschleif Masszahlen gewonnen werden, die gut miteinander verglichen werden koennen. Solche Masszahlen sind beispielsweise Laengen von Differenzvektoren, Flaechen von Projektionen der Vektorschleifen auf Ebenen, Absolute Betraege der Vektoren sowie die zeitliche Funktion dieser Betraege, Vektorzeitflaechen, Vektor-Betragszeitflaechen usw. Mit einem Prozessorsystem koennen diese Masszahlen auch verknuepft werden, was wiederum weitere Aussagen erlaubt.

Im folgenden wird die Erfindung anhand von lediglich einen Ausfuehrungsweg darstellenden Zeichnungen naeher erlaeutert. Es zeigt:

Figur 1 eine schematische Darstellung mehrerer Vektoren im Raum,

Figur 2 Schleifen, die Spitzen von Vektoren beschreiben,

Figur 3 mehrere Vektoren mit ihren Differenzvektoren,

Figur 4 absolute Betraege der Differenzvektoren gemaess Figur 3,

Figur 5 Schleifen und deren Projektionen auf eine Ebene,

Figur 6 eine Betragsfunktion der Vektoren,

Figur 7 eine schematische Darstellung der Vorrichtung und

Figur 8 einen Teil der Vorrichtung gemaess Figur 7.

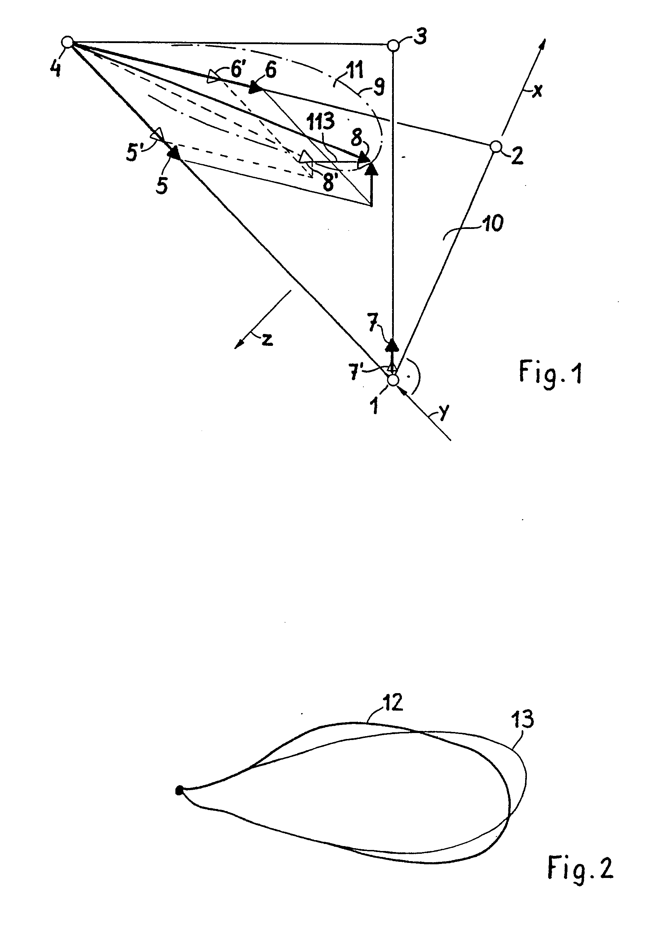

Figur 1 zeigt vier Positionen 1, 2, 3 und 4 fuer Elektroden in einem physiologisch erzeugten Feld wie es ein nicht naeher dargestelltes Lebewesen, das beispielsweise ein Mensch ist, erzeugt. An den Positionen 1 bis 4 werden elektrische Potentiale erfasst. Potentialdifferenzen zwischen je zwei Positionen sind durch Teilvektoren 5, 5', 6, 6' und 7, 7' dargestellt. Die Teilvektoren 5, 6 und 7 sind dabei zu einer bestimmten ersten Zeit und die Teilvektoren 5', 6' und 7' sind zu einer bestimmten zweiten Zeit ermittelt. Durch Vektoraddition erhaelt man den Vektor 8 zur ersten Zeit und den Vektor 8' zur zweiten Zeit. Eine strichpunktiert gezeichnete Schleife 9 zeigt den geometrischen Ort, den die Spitze des Vektors 8, 8' zu aufeinanderfolgenden Zeiten einer Periode einnimmt. Die Positionen 1, 2 und 4 definieren eine Ebene 10, die im wesentlichen parallel zu einer Flaeche 11 ist, die durch die Schleife 9 definiert wird. Die Flaeche 11 ist aber im allgemeinen nicht notwendigerweise eben. Ferner sind Achsen x, y und z fuer ein rechtwinkliges Koordinatensystem eingezeichnet. Dabei steht die z-Achse senkrecht auf einer Ebene die durch die x-und die y-Achse aufgespannt wird.

Figur 2 zeigt Schleifen 12 und 13 die zu verschiedenen Zeiten vom gleichen Feld eines Lebewesens oder von verschiedenen Exemplaren eines Lebewesens aufgenommen wurden.

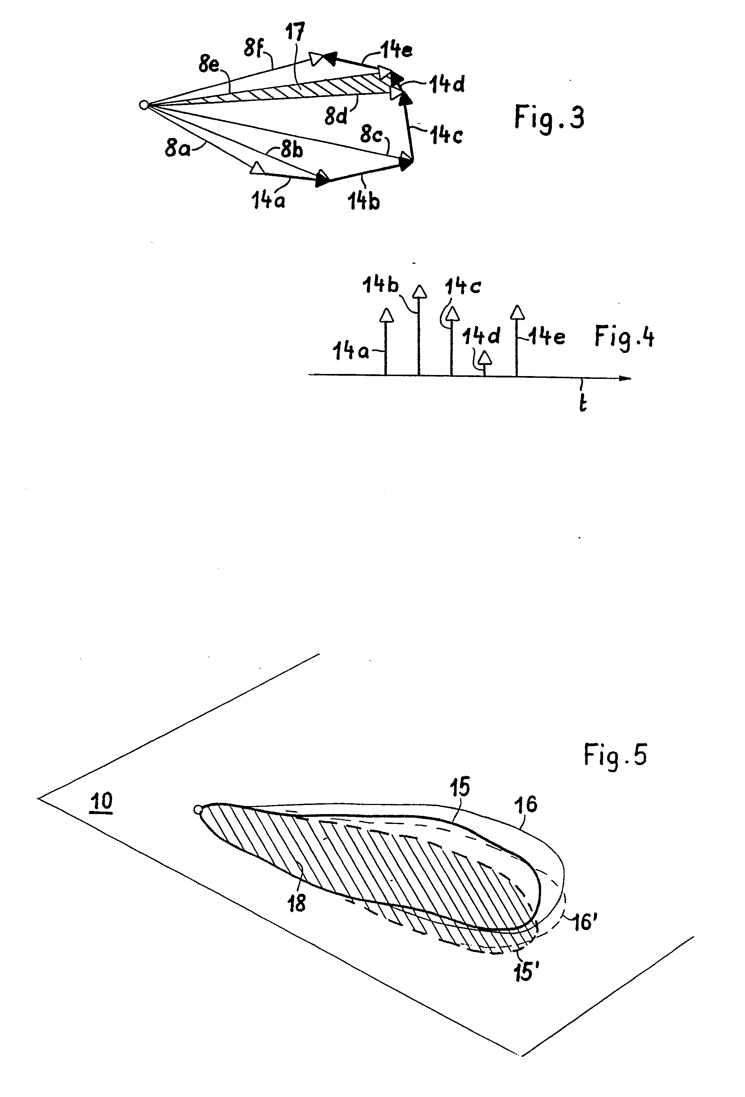

Figur 3 zeigt Vektoren 8a, 8b, 8c, 8d, 8e und 8f die beispielsweise jeweils nach gleichen Zeitabschnitten in einer Periode erfasst wurden. Als Periode verstehen wir einen Zeitabschnitt, in welchem immer wieder dieselben physikalischen Vorgaege ablaufen. In diesem Zusammenhang ist eine Periode beispielsweise gleichzusetzen mit einem Herzschlagintervall. Differenzvektoren 14a, 14b, 14c, 14d und 14e verbinden je zwei aufeinanderfolgende Vektoren 8a, 8b usw. Differenzvektoren sind Groessen, die aus den Vektoren abgeleitet sind und auch ein Mass fuer die Geschwindigkeit, mit der der Vektor 8 sich in den genannten Zeitabschnitten aendert. Zwischen den Vektoren 8d und 8e ist auch eine Vektorzeitflaeche 17 eingezeichnet. Sie gibt an, welche Flaeche der Vektor in einer Zeiteinheit ueberstreicht.

Figur 4 zeigt als weitere Groessen die absoluten Betraege der Differenzvektoren 14a bis 14e beispielsweise in Funktion der Zeit aufgetragen. Die dargestellten Betraege sind dabei beispielsweise innerhalb einer Periode ermittelt worden.

Figur 5 zeigt zwei Schleifen 15 und 16 die den zeitlichen Verlauf des Vektors 8 in verschiedenen Perioden angeben sowie deren Projektionen 15′ und 16′ auf die Ebene 10 gemaess Figur 1. Mit 18 ist die Flaeche innerhalb der Projektion 15′ der Schleife 15 bezeichnet. Die Flaeche 18 wird vom projizierten Vektor in einer Periode ueberstrichen.

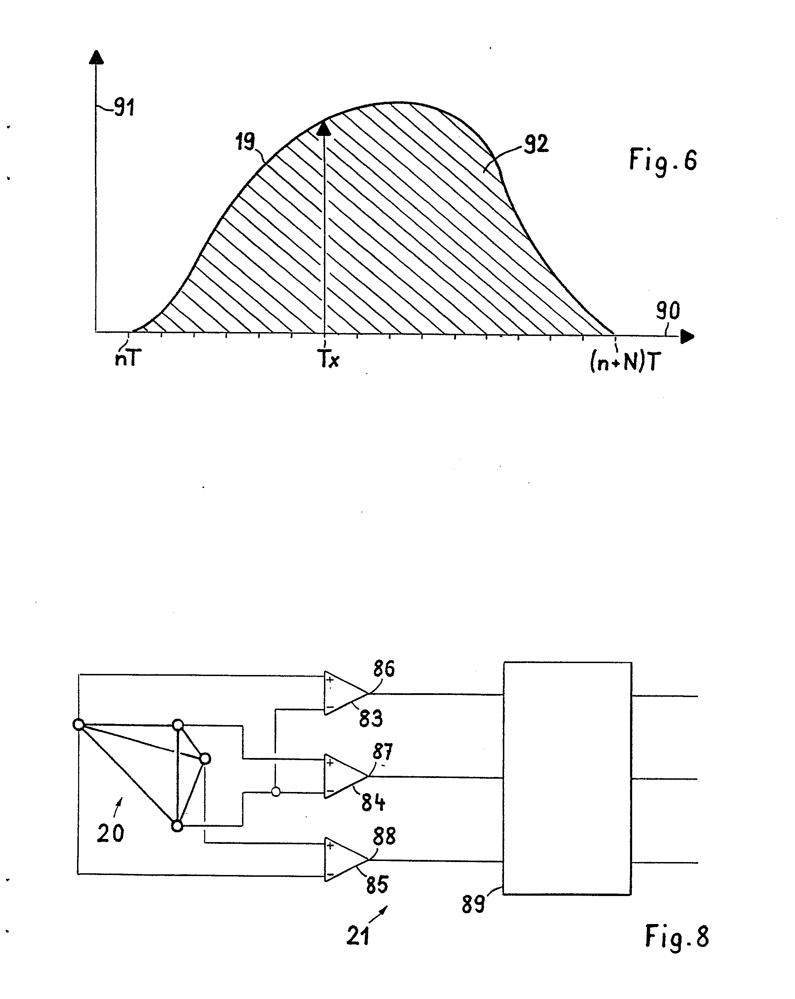

Figur 6 zeigt ein Beispiel einer Betragsfunktion 19 eines Vektors 8, 8′ wie er aus der Figur 1 bekannt ist. Wenn der Vektor 8, 8′ beispielsweise ausgehend von der Position 4 in einer Periode die Schleife 9 durchlaeuft, so folgen die absoluten Betraege des Vektors 8, 8′ der Betragsfunktion 19. Das bedingt, dass die horizontale Achse 90 eine Zeitachse ist und dass die vertikale Achse 91 Werte fuer Betraege des Vektors traegt. Daraus sind die Aenderungen des absoluten Betrages des Vektors ueber die Zeit erkennbar. Die Flaeche 92 unter der Betragsfunktion 19 kann auch als Integral der absoluten Betraege des Vektors ueber eine Periode bezeichnet werden.

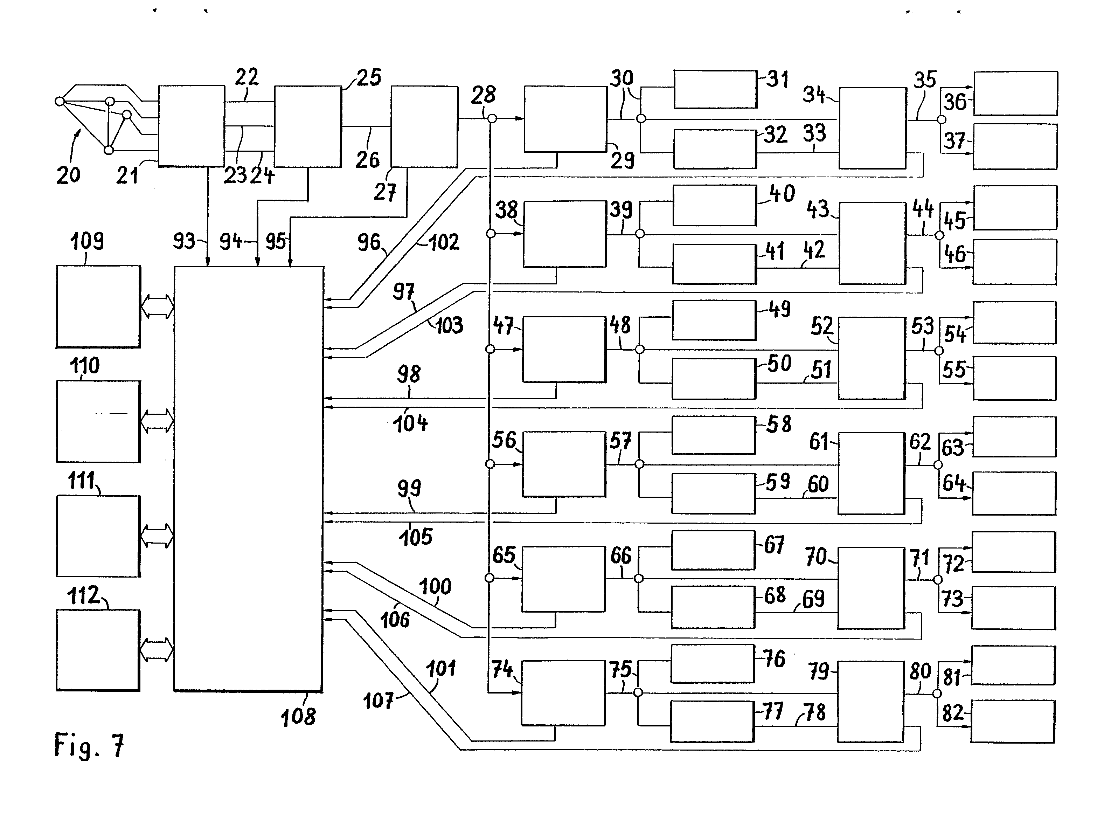

Figur 7 zeigt eine schematische Darstellung der erfindungsgemaessen Vorrichtung. Diese umfasst eine Anordnung 20 fuer Elektroden gemaess Figur 1 mit Leitungen. Die Elektroden greifen in an sich bekannter Weise Potentiale in einem physiologisch erzeugten Feld ab. Diese Elektroden sind an eine Differenzbildungsschaltung 21 angeschlossen, die anhand der Figur 8 genauer beschrieben ist. Die Differenzbildungsschaltung 21 bildet ein Mittel zur Bildung von Potentialdifferenzen und Teilvektoren. Leitungen 22, 23 und 24 verbinden diese mit einer Schaltung 25 zur Addition der Teilvektoren zu einem Vektor, wobe die Teilvektoren ueber die Leitungen 22, 23 und 24 aus der Differenzbildungsschaltung 21 zugefuehrt werden. Solche Schaltungen 21 und 25 koennen durch entsprechend programmierte Mikroprozessoren realisiert werden. Eine Leitung 26 oder ein Bus verbindet die Schaltung 25 mit einer Speicherschaltung 27, die aus einem Speicher besteht, und die es erlaubt die Werte einer Anzahl gespeicherter Vektoren zu speichern, so dass sie fuer weitere Bearbeitungen verfuegbar sind. Dabei sind die Vektoren mit ihren Koordinaten im Koordinatensystem gemaess Figur 1 und der Zeit, zu der die Ausgangswerte, also die Potentiale, abgetastet wurden, gekennzeichnet.

Eine Leitung 28 oder ein Bus verbindet die Speicherschaltung 27 mit einer Einheit 29 zur Bildung von Differenzvektoren. Diese Einheit 29 besteht im wesentlichen aus einem Speicher und aus einem Rechner. Sie ist in der Lage aus dem Betrag und der Richtung zweier Vektoren oder aus den Koordinaten der Spitzen der Vektoren den Differenzvektor 113 (Fig. 1) gemaess den bekannten Regeln der Vektoraddition zu berechnen und dessen Koordinaten im Koordinatensystem mit den Achsen x, y und z anzugeben. Dazu ist der Rechner der Einheit 29 gemaess diesen bekannten Regeln programmiert. Die berechneten Werte fuer den Differenzvektor werden ueber Leitungen 30 an eine Anzeigeeinheit 31 und eine Speichereinheit 32 abgegeben. In der Anzeigeeinheit 31 werden die Werte fuer den Betrag und die Stellung des Differenzvektors in einem Koordinatensystem angezeigt. Dies kann durch die Ausgabe der betreffenden Zahlenwerte ueber einen Drucker und/oder durch graphische Darstellung des Differenzvektors oder aller Differenzvektoren einer Periode miteinander auf einem Bildschirm erfolgen. Die Speichereinheit 32 besteht aus einem RAM und/oder aus einem weiteren Speicher wie beispielsweise einem Plattenspeicher oder einem Bandspeicher. Von der Speichereinheit 32 werden gespeicherte Werte ueber eine Leitung 33 abgegeben und von der Einheit 29 zur Bildung der Differenzvektoren werden aktuelle Werte ueber die Leitung 30 einer Auswerteeinheit 34 zugefuehrt. Diese besteht beispielsweise aus einem Rechner und einem Speicher. Sie kann Differenzen ermitteln zwischen den Werten von Differenzvektoren die zu aufeinanderfolgenden Zeiten einer Periode ermittelt wurden. Sie kann aktuelle Werte mit gespeicherten Werten desselben Subjektes oder mit gespeicherten Werten anderer Subjekte der gleichen Art einander gegenueberstellen und die Abweichung berechnen. Ueber eine Leitung 35 werden die, in der Auswertungseinheit 34 ermittelten, Werte wieder einer Anzeigeeinheit 36 und einer Speichereinheit 37 zugefuehrt. Diese entsprechen den Einheiten 31 und 32. Die Einheit 29 kann auch zur Berechnung der Vektorzeitflaeche 17 (Fig. 3) aus den Vektoren und Differenzvektoren programmiert werden. Beispielsweise kann die Vektorzeitflaeche 17 durch Berechnung der Flaeche eines Dreieckes in an sich bekannter Weise ermittelt werden, wobei das Dreieck durch zwei Vektoren 8d, 8e und den Differenzvektor 14d begrenzt wird.

Die Darstellung und Bildung der Differenzvektoren von Vektoren, die auf das Herz bezogen sind, erleichtert es, elektrophysiologische Vorgaenge wie Depolarisation und Repolarisation des Dipols (welcher das Herz elektrisch darstellt) zu erkennen und besser voneinander zu trennen.

3

Die Leitung 28 ist ebenfalls mit einer Einheit 38 zur Darstellung einer Vektorschleife verbunden. Die Einheit 38 speichert die Koordinaten der Spitzen der Vektoren 8 gemaess Figur 1 im Raum fuer jede Periode. Sie besteht somit aus einem Speicher. Sie kann aber auch durch einen Rechner ergaenzt werden, dem ein Programm fuer die Koordinatentransformation zur Verfuegung gestellt ist. Damit laesst sich der Vektor 8, 8' in weiteren Koordinatensystemen berechnen und im Speicher-speichern. Das bedeutet, dass man die Koordinaten fuer die Vektoren einer Periode gleichzeitig einer Anzeigeeinheit zufuehren kann und dort die Schleife 9 beispielsweise aus verschiedenen Richtungen oder in verschiedenen Projektionen betrachten kann. Ueber Leitungen 39 ist deshalb die Einheit 38 mit einer Anzeigeeinheit 40 und einer Speichereinheit 41 verbunden, die es einerseits erlauben solche Vektorschleifen graphisch oder durch Angabe von Werten darzustellen und andererseits zu speichern. Eine Leitung 42 ist vorgesehen um die Speichereinheit 41 mit einer Auswerteeinheit 43 zu verbinden. Diese ist ueber die Leitungen 39 auch an die Einheit 38 angeschlossen. Die Auswerteeinheit 43 besteht aus einem Rechner und einem Speicher und ermoeglicht den Vergleich einer aktuellen Vektorschleife mit einer gespeicherten alten Vektorschleife. Dies erlaubt es charakteristische Abweichungen der Konturen der Vektorschleifen zu ermitteln. Diese Abweichungen lassen sich durch Gegenueberstellung alter und aktueller Werte oder verschiedener alter Werte aus dem Speicher fuer gewisse gewaehlte Zeitabschnitte ermitteln. Solche Abweichungen geben Hinweise auf den Ablauf von elektrophysiologischen Vorgaengen im Lebewesen. Ueber Leitungen 44 ist die Auswerteeinheit 43 ebenfalls mit einer Anzeigeeinheit 45 und einer Speichereinheit 46 verbunden.

Die Leitung 28 ist weiter mit einer Einheit 47 zur Ermittlung der Flaeche der Projektion einer Vektorschleife in eine Ebene verbunden. Die Einheit 47 besteht aus einem Rechner und einem Speicher und ist ueber Leitungen 48 mit einer Anzeigeeinheit 49 und einer Speichereinheit 50 verbunden. Eine solche zu ermittelnde Flaeche ist in der Figur 5 mit 18 bezeichnet und kann somit in bekannter Weise ausschliesslich unter Verwendung der x-und y-Koordinaten des Vektors 8 ermittelt werden. Dazu werden waehrend einer Periode mehrere Vektoren 8 zu verschiedenen Zeiten ermittelt. Dann werden auch die Differenzvektoren 14 zu diesen Vektoren 8 ermittelt. Aus je zwei Vektoren und dem dazugehoerigen Differnzvektor kann dann die Vektorzeitflaeche berechnet werden. Die Flaeche 18 der Schleife ergibt sich dann durch Addition aller Vektorzeitflaechen. Werden in einer Periode viele Vektoren 8 ermittelt, so erhaelt man daraus die Flaeche 18 mit entsprechend groesserer Genauigkeit. Die Anzeigeeinheit 49 zeigt die Form und/oder den Inhalt der Flaeche 18 an. Die Speichereinheit 50 speichert die Werte zur Form und/oder zum Inhalt der Flaeche 18. Eine Leitung 51 verbindet die Speichereinheit 50 mit einer Auswerteeinheit 52, die vorausgehend gemessene und gespeicherte Flaechen mit der aktuellen Flaeche vergleicht. Der Vergleich des Wertes oder der Groesse dieser Flaechen erlaubt es ebenfalls Schluesse ueber die Arbeit des Organismus des Lebewesens zu ziehen. Leitungen 53 verbinden diese Auswerteeinheit 52 mit einer Anzeigeeinheit 54 und einer Speichereinheit 55. Diese zeigen bzw. speichern Flaechendifferenzen.

Die Leitung 28 ist weiter mit einer Einheit 56 zur Ermittlung der absoluten Betraege des Vektors 8, 8' verbunden. Die Einheit 56 besteht aus einem Rechner und einem Speicher und ist ueber Leitungen 57 mit einer Anzeigeeinheit 58 und einer Speichereinheit 59 verbunden. Die Einheit 56 berechnet die absoluten Werte des Vektors 8, 8' zu verschiedenen aufeinanderfolgenden Zeiten waehrend einer Periode und zwar in an sich bekannter Weise aus den Quadraten der einzelnen Teilvektoren x, y, z. Beispielsweise betraegt der absolute Betrag des Vektors 8 zur Zeit Tx:

$$|v| = \left| \sqrt{x^2(Tx) + y^2(Tx) + z^2(Tx)} \right|$$

Diese Werte werden auch in der Speichereinheit 59 gespeichert. Eine Leitung 60 verbindet die Speichereinheit 59 mit einer Auswerteeinheit 61. Diese vergleicht gespeicherte Werte mit aktuellen Werten. Auf diese Weise koennen Veraenderungen im Elektrolyt bzw. in den Geweben des Lebewesens, die fuer die Leitung der elektrischen Signale von Bedeutung sind, ermittelt werden. Die Auswerteeinheit 61 ist ueber Leitungen 62 auch mit einer Anzeigeeinheit 63 und einer Speichereinheit 64 verbunden.

Die Leitung 28 ist ferner ebenfalls mit einer Einheit 65 zur Ermittlung der Vektor-Betragszeitflaeche 92 (Fig. 6) verbunden. Die Vektor-Betragszeitflaeche 92 wird in der Figur 3 durch die Betragsfunktion 19 und die Zeitachse 90 begrenzt. Wird angenommen, dass der Vektor 8, 8' zum Durchlaufen seiner Schleife 9 eine Periode benoetigt, die mit (n + N)T-nt bezeichnet wird, so berechnet sich die Vektor-Betragszeitflaeche nach der folgenden Formel:

$$F = \sum_{i=n}^{n+N} \left[ \left| \sqrt{x^2(iT) + y^2(iT) + z^2(iT)} \right| \cdot \left( (i)T - (i-1)T \right) \right]$$

Die Einheit 65 besteht aus einem Rechner und aus einem Speicher. Sie berechnet die obengenannte Flaeche gemaess der Formel und gibt die Resultate ueber Leitungen 66 an eine Anzeigeeinheit 67 und an eine Speichereinheit 68 ab. Diese Flaeche 92 gibt einen Hinweis auf den Energieumsatz der elektrophysiologischen Vorgaenge. Eine Leitung 69 verbindet die Speichereinheit 68 mit einer Auswerteeinheit 70 in der die gespeicherten Werte mit den aktuellen Werten der Flaeche verglichen werden. So lassen sich Abweichungen des gerade gemessenen Exemplares zu frueher gemessenen Werten oder zu Durchschnittswerten ermitteln. Leitungen 71 verbinden die Auswerteeinheit 70 mit einer Anzeigeeinheit 72 und einer Speichereinheit 73.

Die Leitung 28 ist auch mit einer Einheit 74 zur Ermittlung des Vektors zu bestimmten Zeiten verbunden. Diese Einheit 74 besteht ebenfalls aus einem Rechner und einem Speicher und ist ueber Leitungen 75 an eine Anzeigeeinheit 76 und eine Speichereinheit 77 angeschlossen. Diese zeigen und speichern den Betrag und die Richtung des Vektors zu verschiedenen Zeiten. Ueber eine Leitung 78 ist die Speichereinheit 77 mit einer Auswerteeinheit 79 verbunden, die alte Werte mit neuen aktuellen Werten vergleicht. Die daraus folgenden Abweichungen werden ueber Leitungen 80 einer Anzeigeeinheit 81 und einer Speichereinheit 82 zugefuehrt.

Die Schaltungen 21, 25 und 27, die Einheiten 29, 38, 47, 56, 65 und 74, sowie die Auswerteeinheiten 34, 43, 52, 61, 70 und 79 sind ueber Leitungen 93 bis 107 mit einem Prozessorsystem 108 verbunden, welches mit einer Anzeigeeinheit 109, einer zusaetzlichen Speichereinheit 110 und einer Dokumentiereinheit 111 versehen ist. Eine Eingabeeinheit 112 fuer die ganze Vorrichtung ist an das Prozessorsystem 108 angeschlossen und besteht beispielsweise aus einer Tastatur, die durch einen Bildschirm ergaenzt werden kann. Als Prozessorsystem 108 ist ein im Handel erhaeltliches Mikroprozessorsystem (beispielsweise Serie 68000) denkbar. Ebenso ist als Anzeigeeinheit 109 ein bekannter Bildschirm und als Dokumentiereinheit 111 ein Drucker vorzusehen. Dem Prozessorsystem 108 koennen Norm-oder Grenzwerte fuer alle ueber die Leitungen 93 bis 107 verfuegbaren Groessen, dem Betrieb der Vorrichtung vorausgehend, eingegeben werden. Dann kann das Prozessorsystem 108 diese Groessen zusammen mit diesen vorgegebenen Werten an die Anzeigeeinheit 109 und an die Dokumentiereinheit 111 ausgeben, die die errechneten Groessen mit den vorgegebenen Werten zusammen darstellen kann. Das Prozessorsystem 108 kann auch so programmiert werden, dass es mehrere ihm zugefuehrte Groessen und Werte zu weiteren Informationen verarbeiten und ausgeben kann.

Da mehrere der Einheiten 29, 38, 47, 56, 65 und 74 einen Speicher, einen Rechner oder beides aufweisen, ist es denkbar, nur einen Rechner und nur einen Speicher vorzusehen. Eine weitere aber an sich bekannte Programmsteuerung sorgt in diesem Falle dafuer, dass jeweils die gewuenschten Rechenprogramme ablaufen. Ebenso koennen mehrere oder gar alle Anzeigeeinheiten in einer Anzeigeeinheit zusammengefasst werden. Dasselbe gilt fuer die Speichereinheiten und die Leitungen, die beispielsweise durch einen Bus ersetzt werden koennten.

Figur 8 zeigt die Anordnung 20 mit der daran angeschlossenen Differenzbildungsschaltung 21. Diese weist drei Differenzverstaerker 83, 84 und 85 auf, deren Ausgaenge 86, 87 und 88 mit einem Mikroprozessor 89 verbunden sind. In den Differenzverstaerkern 83, 84 und 85 werden Differenzen der Potentiale, die an den Positionen 1 bis 4 erfasst werden, ermittelt. Diese werden im Mikroprozessor 89 zu Teilvektoren in einem bestimmten rechtwinkligen Koordinatensystem x, y, z umgewandelt.

## Ansprüche

1. Verfahren zur Verarbeitung von elektrischen Signalen, die als Potentiale in einem physiologisch erzeugten Feld abgegriffen werden, dadurch gekennzeichnet, dass aus den gemessenen Potentialen ein Vektor (8, 8') gebildet wird, der den momentanen Zustand mindestens eines Dipols darstellt, welcher als Modell einer physiologischen elektrischen Aktivitaet verwendet wird, dass der Vektor zu verschiedenen Zeiten ermittelt wird und dass aus mindestens zwei Vektoren weitere Groessen abgeleitet werden.

2. Verfahren nach Anspruch 1, dadurch gekennzeichnet, dass als weitere Groesse ein Differenzvektor (14a) gebildet wird, der zwei Vektoren (8a, 8b) verbindet, die zu verschiedenen Zeiten ermittelt werden.

3. Verfahren nach Anspruch 2, dadurch gekennzeichnet, dass als weitere Groesse der absolute Betrag des Differenzvektors (14a) ermittelt wird.

4. Verfahren nach Anspruch 2, dadurch gekennzeichnet, dass als weitere Groessen Aenderungen des Betrages zwischen aufeinanderfolgenden, in einer Periode ermittelten, Differenzvektoren (14a, 14b, 14c, 14d, 14e) ermittelt werden.

5. Verfahren nach Anspruch 1, dadurch gekennzeichnet, dass der Vektor innerhalb einer Periode mehrmals ermittelt und auf eine Ebene (10) projiziert wird und dass als weitere Groesse eine Flaeche (18), die der projizierte Vektor in einer Periode ueberstreicht, ermittelt wird.

6. Verfahren nach Anspruch 5, dadurch gekennzeichnet, dass die Flaeche (18) mit entsprechenden Flaechen aus anderen Perioden verglichen wird.

7. Verfahren nach Anspruch 1, dadurch gekennzeichnet, dass als weitere Groessen Flaechen (17) ermittelt werden, die zwischen je zwei Vektoren (8a, 8b, 8c, 8d, 8e, 8f) liegen.

8. Verfahren nach Anspruch 7, dadurch gekennzeichnet, dass die Vektoren gleiche Zeitabstaende zueinander aufweisen.

9. Verfahren nach Anspruch 1, dadurch gekennzeichnet, dass als weitere Groesse das Integral (92) der absoluten Betraege der Vektoren (8) ueber eine Periode gebildet wird.

10. Verfahren nach Anspruch 9, dadurch gekennzeichnet, dass Integrale aus verschiedenen Perioden verglichen werden.

11. Verfahren nach Anspruch 1, dadurch gekennzeichnet, dass die weiteren Groessen in verschiedenen Perioden ermittelt und miteinander verglichen werden.

12. Vorrichtung zur Verarbeitung von elektrischen Signalen nach Anspruch 1, gekennzeichnet durch Mittel zur Messung von Potentialen, durch Mittel zur Bildung eines Vektors aus gemessenen Potentialen zu verschiedenen Zeiten und durch Mittel zur Ermittlung weiterer Groessen aus den Vektoren.

13. Vorrichtung nach Anspruch 12, dadurch gekennzeichnet, dass die Mittel zur Ermittlung weiterer Groessen zur Ermittlung von Differenzvektoren ausgebildet sind.

14. Vorrichtung nach Anspruch 12, dadurch gekennzeichnet, dass die Mittel zur Ermittlung weiterer Groessen, zur Ermittlung von Flaechen zwischen Projektionen des Vektors auf eine Ebene ausgebildet sind.

15. Vorrichtung nach Anspruch 12, dadurch gekennzeichnet, dass die Mittel zur Ermittlung weiterer Groessen, zur Bestimmung des absoluten Betrages des Vektors und zum Integrieren dieser Betraege ueber eine Periode ausgebildet sind.

16. Vorrichtung nach Anspruch 12, dadurch gekennzeichnet, dass als Mittel zur Ermittlung weiterer Groessen, Rechner und Speicher vorgesehen sind.

Fig.1

Fig.2

Fig.3

Fig.4

Fig.5

91

19

92

Fig.6

nT

Tx

(n+N)T

90

86

83

87

84

88

85

20

21

89

Fig.8

Fig. 7

0 278 215

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,X | EP-A-0 086 429  (SANZ) <br> * Figuren 1-4, 7a,b,9,11; Seite 9, Zeile 4 - Seite 24, Zeile 18; Seite 32, Zeile 13 - Seite 37, Zeile 14; Seite 42, Zeilen 1-16 * & US-A-4 569 357 (Kat. D) | 1,12 | A 61 B    5/04 |
| A | | 6,14,16 | |
| A | DE-A-2 719 803  (HEALTH TECHNOLOGY LABORATORIES, INC.) <br> * Figuren 1,6,7; Seite 12, Zeile 6 - Seite 13, Zeile 10; Seite 15, Zeile 21 - Seite 24, Zeile 10; Seite 25, Zeile 8 - Seite 31, Zeile 20 * <br> --- | 1,12,14 | |
| A | COMMUNICATIONS OF THE ACM, Band 5, Nr. 2, Februar 1962, Seiten 118-122, New York, US; G.E. FORSYTHE et al.: "Vectorcardiographic diagnosis with the aid of ALGOL" <br> * Seiten 118-120, Abschnitt "Physiological basis of vectorcardiography"; Figuren 1-4 * <br> --- | 1 | |
| | | | **RECHERCHIERTE SACHGEBIETE (Int. Cl.4)** |
| A | EP-A-0 150 352  (WILLI STUDER AG) <br> * Figuren 1,2; Seite 3, Zeile 17 - Seite 7, Zeile 15 * <br> ---                         -/- | 1,2,12 | A 61 B <br> G 06 F |

Der vorliegende Recherchenbericht wurde für alle Patentansprüche erstellt

| Recherchenort | Abschlußdatum der Recherche | Prüfer |
|---|---|---|
| DEN HAAG | 29-04-1988 | CHEN A.H. |

EPO FORM 1503 03.82 (P0403)

### Europäisches Patentamt

## EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | ELECTRONICS AND COMMUNICATIONS IN JAPAN, Band 57-C, Nr. 3, 1974, Seiten 86-92; A. NISHIYAMA et al.: "An automatic heart-disease diagnosis system using an MOS-IC'ed learning machine" <br> * Seite 86, Abschnitt: "Introduction"; Seiten 87-90, Abschnitte 2.2.1.: "Features of VCG's", 2.2.3: "Identification of ECG waveform", 2.2.4.: "Clinical test results"; Figuren 1-13 * <br> --- | 1,6,12, 14,16 | |
| P,X | EP-A-0 223 049 (KESSLER) <br> * Figuren 1a,b; Seite 6, Zeile 19 - Seite 9, Zeile 27 * | 1,12 | |
| P,A | ----- | 16 | |
| | | | RECHERCHIERTE SACHGEBIETE (Int. Cl.4) |

Der vorliegende Recherchenbericht wurde für alle Patentansprüche erstellt

| Recherchenort | Abschlußdatum der Recherche | Prüfer |
|---|---|---|
| DEN HAAG | 29-04-1988 | CHEN A.H. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
................................................................
& : Mitglied der gleichen Patentfamilie, übereinstimmendes Dokument

EPO FORM 1503 03.82 (P0403)